# EUROPEAN PATENT APPLICATION

(11) **EP 3 750 407 A1**
(43) Date of publication of application: **16.12.2020**
(21) Application number: 19748181.5
(22) Date of filing: 29.01.2019
(51) Int. Cl.: A23J 3/14, C12N 5/04, C12N 9/14

(54) **METHOD FOR OBTAINING TRANSFORMED PLANT CELLS**

(30) Priority: 05.02.2018 RU 2018104401
(71) Applicant: Obshestvo s Ogranichennoi Otvetstvennostju "Innovative Pharmacology Research" ("IPHAR"), Tomsk 634021 (RU)
(72) Inventor: S HMYKOVA, Natalya Anatolyevna, Tomsk, 634021 (RU); KOMAKHIN, Roman Alexandrovich, Moscow, 117216 (RU); STANKEVICH, Sergey Aleksandrovich, Tomsk, 634062 (RU); KHAZANOV, Veniamin Abramovich, Tomsk, 634034 (RU)
(74) Representative: Jeck, Anton
(86) International application number: PCT/RU2019/000053
(87) International publication number: WO 2019/151902

(57) **Abstract**

The invention relates to food industry and medicine and describes the method of producing transformed plant cells, containing recombinant human alkaline phosphatase, and their use for maintaining the homeostasis of gastrointestinal tract as an agent, regulating gastrointestinal tract microflora and the immune system.

A method of producing transformed plant cells, containing recombinant human alkaline phosphatase, comprising the creation of a plant expression vector with human alkaline phosphatase gene, introduction of the plant expression vector with human alkaline phosphatase gene into agrobacterial strains, production of plant callus cells, agrobacterial transformation of callus cells using agrobacterial strains; production of somatic embryos from the transformed callus cells and growing the somatic embryos, containing human alkaline phosphatase gene, in a suspension culture.

A variant of the method of producing transformed plant cells, containing recombinant human alkaline phosphatase, is the method in which after agrobacterial transformation of callus cells, the transformed callus cells with human alkaline phosphatase gene are grown in a suspension culture.

The produced transformed plant cells may be used to regulate gastrointestinal tract microflora, to prevent immune system disorders and restore a disrupted immune system as an immunomodulating agent.

## Description

### FIELD OF THE INVENTION

The invention relates to food industry and medicine and describes the method of producing transformed plant cells, containing recombinant human alkaline phosphatase, and their use for maintaining the homeostasis of gastrointestinal tract (GI tract).

### DESCRIPTION OF THE PRIOR ART

It is well known that the intestine is an important barrier organ, consisting of normal (synanthropic) microflora, mucosal layer, epithelium and subepithelial immune system. The main function of the intestinal barrier is protecting the organism from bacterial translocation into systemic circulation. Normal microflora can stay in intestinal lumen without stimulating the host's immune response, however, the same bacteria can induce immune response and inflammation if they translocate through the intestinal barrier into the blood and get transported into other organs. Disruption of the intestinal barrier leads to the development of excessive immune response to the components of synantropic bacteria, and, consequentially, to the development of chronic inflammatory bowel diseases (IBD), such as Crohn's disease and nonspecific ulcerative colitis (Podolsky, 2010). Stress, infections, aging and toxic action of xenobiotics, including medicines, also play an important part in the development of these disorders (Cadwell et al., 2010).

Currently, the only widely available means of maintaining gastrointestinal tract homeostasis, including regulating GI tract microflora and the immune system, are drugs and products based on probiotics, live microorganism cultures composed of species normally present in the intestine. However, there is no convincing evidence of clinical efficacy of such products. Moreover, externally introduced microflora usually doesn't survive in the human organism and is rejected by the naturally present microflora. Also, probiotics are usually 2 types of bacteria: Lactobacillus and Bifidobacterium, while human microflora includes over 300 types of bacteria. So after stopping the use of probiotics the initial disorders of gastrointestinal tract homeostasis usually recur.

However, new methods of GI tract homeostasis regulation, based on the use of the alkaline phosphatase (AP) enzyme, are considered promising, because disorders of microflora, immune system and intestinal barrier function, as well as IBD development, are in large part initiated by inflammation inducers, some of which are physiological substrates of the AP enzyme. The most potent inflammation inducers are: 1) lipopolysaccharides (LPS) - bacterial endotoxins of E.coli and other Gram-negative bacteria; 2) extracellular ATP and other nucleotides and nucleosides. LPS, which is constantly released by intestinal microflora, gets into blood when the intestinal barrier is disrupted, and induces inflammation even in minimal (ng/kg) concentrations. During this process, LPS binds to membrane protein CD14 on the surface of macrophages, leading to their activation, which in turn leads to the expression of dozens of biologically active compounds: prostaglandins, nitric oxide and cytokines, including interleukins and TNFα (Nathan, 1987). LPS and extracellular ATP are very important factors of the development of systemic immune and inflammatory response to «stranger» and «danger» signals (Matzinger, 2002).

Intestinal alkaline phosphatase, which is produced by the cells of intestinal mucosa, plays an important part in intestinal homeostasis by deactivating (detoxifying) LPS and preventing LPS translocation, regulating intestinal microflora and pH of intestinal surface (Eskandari et al., 1999; Weemaes et al., 2002; Riggle et al., 2013; Tuin et al., 2009). LPS dephosphorylation, catalyzed by AP, eliminates its proinflammatory activity and thus protects the organism from the development of different inflammatory and autoimmune diseases (Park, Lee, 2013).

Disruption of normal level (activity) of intestinal AP due to reduced expression of genes, which code AP, is observed in inflammatory bowel diseases, coeliac disease and obesity. Oral administration of AP to mice with colitis reduces the mRNK level of proinflammatory cytokine TNFα and nitric oxide (Muginova et al., 2007). Molnar et al (2012) have demonstrated that AP levels in inflamed intestinal mucosa of children with Crohn's disease and nonspecific ulcerative colitis are significantly lower than in control group. Administration of exogenous AP to patients with active form of intestinal inflammation may prevent worsening of the disease. It has been demonstrated in another study that administration of exogenous AP has reduced bowel inflammation in newborn animals, so it was proposed that AP may be used to prevent inflammatory diseases in newborn babies (Biesterveld et al., 2015).

Several prior art patents and patent applications exist, describing the use of AP for regulating intestinal microflora, immune system activity, prevention and treatment of different diseases: US20130280232 dated 24.10.2013 «Use of alkaline phosphatase for the detoxification of LPS present at mucosal barriers», US20110206654 dated 25.08.2011 «Methods of Modulating Gastrointestinal Tract Flora Levels with Alkaline Phosphatase», US20130022591 dated 24.01.2013 «Methods of Reducing or Inhibiting Toxic Effects Associated with a Bacterial Infection Using Alkaline Phosphatase», US8574863 dated 05.11.2013 «Alkaline phosphatase for treating an inflammatory disease of the gastrointestinal tract».

The closest method to the method of food product production, disclosed in the present invention, is the method disclosed in patent application US20110206654 dated 25.08.2011 «Methods of Modulating Gastrointestinal Tract Flora Levels with Alkaline Phosphatase», describing the use of AP to regulate GI tract microflora, including for protection and restoration of normal microflora during the use of antibiotics. In this prototype and other published sources, the authors have used AP as a pure protein, purified from extraneous proteins and other impurities. This AP was administered to animals or humans (in clinical trials) as a pure protein or as a pharmaceutical formulation, containing purified AP and different excipients. The purified AP was obtained by different methods, including: isolation of AP from bovine intestinal mucosa; isolation of AP from mammal placenta: production of recombinant human AP in mammal cell culture with subsequent isolation and purification. A common feature of the known methods is production of AP as a pure protein, which leads to several disadvantages, listed below, and practically eliminates the possibility of its use as a food product or a food component.

A very important disadvantage of AP, isolated from mammal intestine or other organs, is that it's a foreign protein to humans (due to interspecies genetic differences), so its use in humans may induce sensitization and the development of dangerous allergic reactions after repeated use. Besides, AP content in animal tissues is low, requiring the costly process of protein extraction and purification. There is also a risk of contamination with prions and pathogenic microorganisms. Thorough purification greatly increases the cost of end products and severely limits its practical use.

Production of recombinant human AP in mammal cell systems *in vitro* has its own disadvantages, including: limited scalability of the process, high cost and risk of contamination with human pathogens.

A common disadvantage of the described methods of AP production as a pure protein is the high cost of the end product (up to 1000-2000 USD per 1 package), due to the difficulty of isolation, purification and quality control of recombinant protein. This practically eliminates the possibility of its use as a food product for prevention and treatment of disorders.

Another common disadvantage of the described methods of AP production as a pure protein is the high sensitivity of AP to external conditions, such as temperature and pH (acidity) of the medium. For example, AP becomes completely inactivated in acidic medium of the stomach. This eliminates the possibility of using such AP in food products, or requires the creation of complex dosage forms, resistant to stomach acid.

The aforementioned disadvantages of AP production preclude the use of AP as a widely available product for maintaining normal intestinal microflora and immune system and for preventing inflammatory and autoimmune diseases, associated with the disruption of gastrointestinal tract homeostasis. Therefore, the possibility of using AP as a pure protein or as a mixture with standard pharmaceutical excipients for regulating gastrointestinal tract microflora, described in the prototype (US20110206654 dated 25.08.2011) is severely limited by the aforementioned disadvantages and can't be used in practice for a food product or food component.

### DETAILED DESCRIPTION OF THE PRESENT INVENTION

The goal of the present invention is to develop a new, effective, safe and affordable food product, containing AP, for maintaining normal intestinal microflora and immune system and for preventing the development of inflammatory and autoimmune diseases, associated with the disruption of gastrointestinal tract homeostasis.

The goal is met by the development of food product, in which recombinant human alkaline phosphatase is located in plant cells. AP-containing plant cells (unlike the prototype, where purified AP is used) provide natural protection from AP inactivation in acidic gastric medium and its delivery into the intestine, where AP performs its function - dephosphorylates (inactivates) bacterial endotoxins, produced by pathogenic microflora of the GI tract. Thus, the AP-containing food product maintains normal GI tract microflora and immune system, prevents disorders of normal GI tract microflora and immune system, restores normal GI tract microflora and immune system in toxic and stress conditions, and also prevents the development of inflammatory and autoimmune diseases. AP-containing plant cells contained in the food product are safe for human consumption, do not induce sensitization and dangerous allergic reactions in repeated use, and can't infect humans with prions and animal pathogenic microorganisms (unlike mammal organ-derived AP). Finally, a food product based on plant cells, containing recombinant human AP, is not only safer but also 2-3 orders of magnitude cheaper than drugs based on purified recombinant human AP produced in mammal cells. This allows to use it as a food product for mass prevention of disorders of GI tract microflora and immune system, and to prevent inflammatory and autoimmune bowel diseases in the general population.

The method of producing a food product based on plant cells, containing recombinant human alkaline phosphatase, comprises the creation of a plant expression vector with human AP gene, introduction of the plant expression vector with human AP gene into agrobacterial strains, production of plant callus cells, agrobacterial transformation of callus cells using agrobacterial strains; the food product is produced by growing the transformed plant callus cells, containing human AP gene, in a suspension culture.

Another variant of the method of producing a food product based on plant cells, containing recombinant human alkaline phosphatase, comprises the creation of a plant expression vector with human AP gene, introduction of the plant expression vector with human AP gene into agrobacterial strains, production of plant callus cells, agrobacterial transformation of callus cells using agrobacterial strains and production of somatic embryos from the transformed callus cells; the food product is produced by growing the somatic embryos, containing human AP gene, in a suspension culture.

During somatic embryos growing, the embryos' development is synchronized by filtering them through sieves of different sizes, centrifuging, automatic separation or other synchronization method. Somatic embryos are grown in a liquid nutrient medium with addition of compounds that increase the osmotic pressure. Optimal concentration of such compounds is no more than 10 percent. Polyethylene glycol, mannitol and other compounds may be used as compounds increasing the osmotic pressure.

Tissue-specific alkaline phosphatases, e.g. intestinal and placental, or tissue-nonspecific alkaline phosphatases, as well as other isoforms of alkaline phosphatase, may be used as the recombinant human alkaline phosphatase for the present invention.

Transformed callus cells or somatic embryos, grown in a suspension culture, are dried and used as a food product, for example, as capsules, tablets, sachet bags and other consumption-ready forms, or by adding them to other food products.

The produced food product is used to regulate GI tract microflora; to prevent immune system disorders and to restore a disrupted immune system as an immunomodulating agent.

It is known, that plant producers are the most promising system for producing high quality, safe and relatively inexpensive proteins (Conley et al., 2011; Sharma, Sharma, 2009; Sabalza et al., 2014). We have used the advantages of plant producers to create a fundamentally novel product, which comprises plants containing recombinant human alkaline phosphatase, instead of pure AP. Any edible plants, usable for producing recombinant proteins, may be used as for this purpose, including, but not limited to, carrot (*Daucus carota*)*,* lettuce (*Latuca sativa*), cabbage (*Brassica oleracea*), Chinese cabbage (*Brassica pekinensis*), dill (*Anethum graveolens*), celery (*Apium graveolens*), cucumber (*Cucumis sativus*), squash (*Cucurbita pepo*), stevia (*Stevia rebaudiana*), tobacco (*Nicotiana tabacum*), rice (*Oryza sativa*), medicago (*Medicago sativa*), tomato (*Solanum lycopersicum*) and other plants. It's preferable to use the plants in which somatic embryogenesis is possible in at least 60% of cells. The plants may be used whole or as components, including, but not limited to, cells, embryos, leaves, stems and other constituents. The plants or their components, containing recombinant human AP, may be consumed intact or ground (even into separate cells), in raw and dried form, using different drying methods or not. The food product may contain tissue-specific AP (e.g. intestinal, placental) or tissue-nonspecific AP or other human alkaline phosphatase.

### The disclosed method (its variants) comprises the following.

The first variant of the method comprises the following steps: creation of a plant expression vector with human AP gene, introduction of the plant expression vector with human AP gene into agrobacterial strains, production of plant callus cells, agrobacterial transformation of callus cells using agrobacterial strains. The food product is produced by growing the transformed plant callus cells, containing human AP gene, in a suspension culture.

The second variant of the method comprises the following steps: creation of a plant expression vector with human AP gene, introduction of the plant expression vector with human AP gene into agrobacterial strains, production of plant callus cells, agrobacterial transformation of callus cells using agrobacterial strains and production of somatic embryos from the transformed callus cells. The food product is produced by growing the somatic embryos, containing human AP gene, in a suspension culture.

The plant cells, containing recombinant human alkaline phosphatase, can also be used for production and growing of plants. The resulting plant biomass may be used as raw material for producing a food product based on plant cells, containing recombinant human alkaline phosphatase.

Creation of a plant expression vector with human AP gene was performed as follows:
Nucleotide sequence of recombinant gene of human AP with a size of 1587 base pairs was created by synthesis in full conformity with the nucleotide sequence of native mRNA of the human alkaline phosphatase gene (Homo sapiens alkaline phosphatase, intestinal (ALPI), Sequence ID: NM_001631.4). To facilitate cloning, a sequence of restriction site B*gl*II (agatct) was added into the sequence of recombinant AP gene at 5'-end before the translation initiation site (atg), while a sequence with the restriction site *Xba*I (atctagaat) was added at 3'-end after the stop codon (tga). Nucleotide sequence of recombinant gene of human AP with a size of 1602 base pairs, containing the sequence of restriction sites *Bgl*II and *Xba*I*,* was cloned into a plasmid pAL-T vector and codenamed pAL-T-AP. Nucleotide sequence of AP gene with a size of 1600 base pairs was removed from pAL-T-AP plasmid at restriction sites *Bgl*II and *Xba*I and ligated into an earlier created plasmid p35S-NLS-*rec*A-*lic*BM3 [1], which was hydrolyzed at restriction sites *Bam*HI and *Xba*I in advance, producing plasmid p35S-AP, in which the recombinant AP gene is under the control of 35S promoter of cauliflower mosaic virus [2]. The accuracy of assembly of genetic construction p35S-AP was verified by sequencing.

Introduction of the plant expression vector with human AP gene into agrobacterial strains was performed as follows:
Introduction of the plant expression vector with human AP gene into agrobacterial strains was performed using the method of «three-parent hybridization», using *Escherichia coli* cells with p35S-AP plasmid as donors, *E. coli* cells of HB101 pRK2013 strain as a facilitator of conjugative transfer and *Agrobacterium tumefaciens* cells of strain GV3101 or strain AGLO as acceptors. This resulted in the production of agrobacterial strains GV3101 p35S-AP and AGLO p35S-AP, containing human AP gene.

Production of plant callus cells and agrobacterial transformation of callus cells.

Plant seeds were sterilized in sterile conditions (laminar flow cabinet) in an aqueous solution of a chlorine-containing commercial disinfectant with the addition of Tween-20 (1 drop per 100 ml of solution), then washed three times in sterile distilled water for 10 minutes each time. The embryos were then extracted from the sterilized seeds in sterile conditions, which were then transferred into vials with modified Murashige and Skoog medium (MSM), enriched with growth regulators 2,4-Dichlorophenoxyacetic acid (2,4-D) and kinetin. The vials were placed into thermostat and incubated in darkness at 23 °C until the callus formed.

**Table 1 - Composition of modified Murashige and Skoog medium (Mutsuda et al., 1981)**

| Components | Concentration in the medium, mg/L | |
|---|---|---|
| NH₄NO₃ | 412,5 | |
| KNO₃ | 2496,3 | |
| CaCl₂ | 332,2 | |
| MgSO₄ × 7H₂O | 370,0 | |
| KH₂PO₄ | 170,0 | |
| Iron chelate | | |
| Na₂ 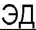 TA ×2H₂O | 37,3 | |
| FeSO₄× 7H₂O | 27,4 | |

| Microelements | | |
|---|---|---|
| H₃BO₃ | 6,200 | |
| MnSO₄ × 5H₂O | 24,100 | |
| ZnSO₄ × 7H₂O | 10,600 | |
| Kl | 0,830 | |
| Na₂MoO₄ × 2H₂O | 0,250 | |
| CuSO₄ × 5H₂O | 0,025 | |
| CoCl₂ × 6H₂O | 0,025 | |

| Organic compounds | | |
|---|---|---|
| Thiamine chloride | 3,0 | |
| Pyridoxine chloride | 0,5 | |
| Nicotinic acid | 5,0 | |
| Meso-inositol | 100,0 | |
| Casein hydrolysate | 500,0 | |
| Sucrose | 20000,0 | |
| Agar | 7000,0 | |

In a laminar flow cabinet, separate bacterial colonies from a fresh culture of agrobacterial strains GV3101 p35S-AP or AGLO p35S-AP with human AP gene were put into a vial (using a sterile inoculation loop) containing 3 of sterile liquid medium LB with antibiotics, corresponding to the bacterial strain. The agrobacteria were grown for 20-48 hours at 28 °C on a shaker with circular rotation (amplitude 5-10 cm and rate 150-200 RPM) equipped with thermostat.

Standard medium was used for growing agrobacteria, for example, LB medium, containing (per 1 L): tryptone - 10 g, yeast extract - 5 g, sodium chloride - 5 g and bacteriological agar - 15 g. The medium was autoclaved in standard conditions for 15-20 min. After cooling to 65 °C antibiotics were added: for strain AGLO - kanamycin and rifampicin, each to final concentration 100 mg/L; for strain GV3101 - kanamycin and rifampicin, each to final concentration 100 mg/l and gentamicin to final concentration 25 mg/L.

Plant callus cells were placed on sterile filter paper in Petri dishes in a laminar flow cabinet, and 10-25 mcl of overnight culture of AGLO agrobacteria and GV3101 with alkaline phosphatase gene were applied on each transplant. After agrobacterial culture application the callus was slightly dried and transported into vials onto MSM nutrition medium with 0.2 mg/L of 2,4-D. After 3 days the calluses were transported on an agar medium with the same composition but with the addition of 500 mg/L cefotaxime and 100 mg/L kanamycin. Cultivation was performed for 10 days in darkness at 22-24 °C. Then 1-2 more transfers were performed onto a medium with the same composition until new callus cell colonies appeared. To reproduce them, callus cells were transferred onto MSM nutrition medium with 0.2 mg/L 2,4-D and 200 mg/L cefotaxime. It's possible to keep callus tissue in the culture for unlimited time, periodically dividing it into fragments and planting them onto new medium. Selection of transgenic carrot cells was performed using the ability of alkaline phosphatase to dephosphorylate para-nitrophenylphosphate, producing yellow-colored para-nitrophenol. To produce the required amount of the food product, callus cells with human AP gene were grown in a suspension culture in a liquid nutrition medium of the same composition without agar.

If the 2nd variant of the method of food product production is used, somatic embryos were produced from transformed plant callus cells, which were then grown in a suspension culture.

Production of somatic embryos from transformed callus cells was performed as follows:
To produce somatic embryos, callus cell suspension was cultivated in liquid MSM nutrition medium, containing 0.2 mg/L IAA (Indole-3-acetic acid) and kinetin. The produced embryogenic suspensions contained different pre-embryonic structures as well as separate non-embryogenic cells and cell groups. To obtain a homogenous population of somatic embryos (to synchronize them), the suspension culture was filtered through nylon sieves with mesh size 120 mcm, then through 50 mcm. The cell mass that remained on the second sieve was transferred to a fresh medium to form embryos. On average, up to 70 thousand embryos could be produced from 1 L of the medium.

Production of a dried food product based on plant callus cells or somatic embryos with human AP gene.

Callus cells, containing human intestinal AP gene, were washed by distilled water from the remaining nutrition medium and were freeze dried at -55 °C with finishing drying at +30 °C, not allowing the proteins to denaturate. The activity of alkaline phosphatase in the dried cell mass was determined by the ability of alkaline phosphatase to dephosphorylate para-nitrophenylphosphate. The prepared mass of plant cells, containing AP gene, was packed into capsules, tablets, sachets or other forms and used as a food component.

To better illustrate the abovementioned embodiments, below is the description of the development and optimization of production technology of the food products.

Plant cells, for example, carrot cells, containing human AP gene, were dried using freeze drying at -55 °C with finishing drying at 20, 30, 40, 50 and 60 °C. Dry biomass (about 1 g) was ground with 10 ml of buffer solution containing 5mM Tris HCI, 0.1 mM magnesium chloride, 0.1 mM zinc chloride, and centrifuged at 100g for 30 min. Dephosphorylating ability of AP in the supernatant was tested by adding 20 mM pNPP (para-nitrophenylphosphate) into the solution. The reaction mixture was incubated at 37 °C for 30 minutes for the reaction products to color the mixture. The reaction was stopped by 2 ml of cooled 0.5 M NaOH. The amount of enzyme required to produce 1 mcM of pNP was taken as the unit of activity. Specific activity was calculated in units per 1 g of carrot cells.

**Table 2 - Activity of AP in carrot cell biomass in different drying modes**

| Item | Temperature mode | Activity, U/q |
|---|---|---|
| 1 | -55 °C, finishing drying +20 °C | 216 |
| 2 | -55 °C, finishing drying +30 °C | 225 |
| 3 | -55 °C, finishing drying +40 °C | 200 |
| 4 | -55 °C, finishing drying +50 °C | 150 |
| 5 | -55 °C, finishing drying +60 °C | 120 |

Table 2 demonstrates that the temperature mode of finishing drying of plant cells affects the activity of AP, the best finishing drying temperatures are in the range of 20 °C to 40 °C.

Effects of nutrition medium composition on callus formation was studied in two cultivars of carrot: Nantskaya 4 and Moscow Winter A-555, belonging to different cultivar groups. Zygotic embryos isolated from mature carrot seeds were used as explants. The embryos were cultivated in three most widely used mediums: MS (Murashige, Skoog, 1964), MSM (Masuda et al, 1981) and B-5 (Gamborg et al., 1976). To induce callus formation, 2,4-D was added to the mediums, the results are presented in Table 3.

**Table 3 - Callus formation in carrot zygotic embryo culture in different nutrition mediums**

| Cultivar | Medium | 2,4-D concentration, mg/L | Percentage of explants with callus, % | Callus biomass, mg/explant |
|---|---|---|---|---|
| Nantskaya 4 | MS | 0.001 | 10 | 200±25 |
| | | 0.1 | 90 | 1400±90 |
| | | 1.0 | 90 | 1700±160 |
| | | 2.0 | 90 | 200±30 |
| | MSM | 0.001 | 15 | 250±18 |
| | | 0.1 | 92 | 2000±150 |
| | | 1.0 | 95 | 1800±200 |
| | | 2.0 | 90 | 350±40 |
| | B-5 | 0.01 | 9 | 150±20 |
| | | 0.1 | 91 | 1000±70 |
| | | 1.0 | 90 | 1100±100 |
| | | 2.0 | 90 | 240±60 |
| Moscow Winter A-555 | MS | 0.01 | 5 | 100±10 |
| | | 0.1 | 80 | 800±50 |
| | | 1.0 | 90 | 1000±90 |
| | | 2.0 | 75 | 200±30 |
| | MSM | 0.01 | 8 | 150±10 |
| | | 0.1 | 85 | 1000±150 |
| | | 1.0 | 90 | 1400±160 |
| | | 2.0 | 88 | 300±20 |
| | B-5 | 0.01 | 5 | 90±10 |
| | | 0.1 | 86 | 700±60 |
| | | 1.0 | 78 | 1000±90 |
| | | 2.0 | 60 | 400±20 |

The table demonstrates that callus formation in carrot happens in all studied mediums, however, the best medium for both carrot cultivars is MSM.

Effects of growth regulators on the capability of plant callus tissues, for example, of carrot of Nantskaya 4 cultivar, to perform somatic embryogenesis were studied in a suspension culture on MSM medium with different combinations of growth regulators like auxins (2,4-D, IAA) and cytokinins (kinetin and benzylaminopurine (BAP)). Callus tissue of Nantskaya 4, produced from zygotic embryos, was used as transplants, the cell suspension was cultivated in 100 ml vials on a shaker at 80 RPM, the results of studies are presented in Table 4.

**Table 4 - Average embryo count, obtained in a suspension culture of Nantskaya 4 in mediums with different combination of growth regulators**

| | Growth regulator | Growth regulator concentration | Embryo count in 10 ml of suspension culture |
|---|---|---|---|
| 1 | 2,4-D | 0.1 | 275±15 |
| | Kinetin | 0.1 | |
| 2 | 2,4-D | 0.1 | 210±20 |
| | BAP | 0.1 | |
| 3 | 2,4-D | 0.2 | 493±35 |
| | Kinetin | 0.2 | |
| 4 | 2,4-D | 0.2 | 286±30 |
| | BAP | 0.2 | |
| 5 | 2,4-D | 1.0 | 175±16 |
| | Kinetin | 1.0 | |
| 6 | 2,4-D | 1.0 | 190±10 |
| | BAP | 1.0 | |
| 7 | 2,4-D | 2.0 | 180±9 |
| | Kinetin | 2.0 | |
| 8 | 2,4-D | 2.0 | 169±10 |
| | BAP | 2.0 | |
| 9 | IAA | 0.1 | 250±20 |
| | Kinetin | 0.1 | |
| 10 | IAA | 0.1 | 200±14 |
| | BAP | 0.1 | |
| 11 | IAA | 0.2 | 450±40 |
| | Kinetin | 0.2 | |
| 12 | IAA | 0.2 | 310±35 |
| | BAP | 0.2 | |
| 13 | IAA | 1.0 | 180±16 |
| | Kinetin | 1.0 | |
| 14 | IAA | 1.0 | 175±19 |
| | BAP | 1.0 | |
| 15 | IAA | 2.0 | 100±12 |
| | Kinetin | 2.0 | |
| 16 | IAA | 2.0 | 90±14 |
| | BAP | 2.0 | |

The table demonstrates that embryo production may satisfactorily occur on mediums not containing 2,4-D.

To produce callus tissue different types of explants were used: taproot tissues, fragments of stem and leaves, petiole, cotyledons, hypocotyl and zygotic embryos of Nantskaya 4 carrot. The explants were cultivated on MSM nutrition medium with 0.2 mg/L 2,4-D, then, in order to induce embryogenesis, the produced callus was transferred on mediums of the following compositions: MSM with 0.2 mg/L 2,4-D and kinetin and MSM with 0.2 mg/L IAA and kinetin. The cell suspension was cultivated in 100 ml vials on a shaker at 80 RPM, the results of studies are presented in Table 5.

**Table 5 - Embryo count produced from callus from different explants of Nantskaya 4 carrot**

| | Explant | Explant count in 10 ml of suspension culture | |
|---|---|---|---|
| | | MSM with 0.2 mg/L 2,4-D and kinetin | MSM with 0.2 mg/L IAA and kinetin |
| 1 | Taproot | 0 | 2±1 |
| 2 | Stem | 20±4 | 15±3 |
| 3 | Leaf | 25±6 | 26±6 |
| 4 | Petiole | 30±7 | 28±6 |
| 5 | Cotyledons | 45±11 | 46±9 |
| 6 | Hypocotyl | 120±13 | 100±11 |
| 7 | Zygotic embryos | 405±16 | 397±15 |

Table 5 demonstrates that zygotic embryos are the best explant for producing embryogenic callus.

The possibility of production and use of food products based on plants, containing human AP, is demonstrated by the following examples.

For convenience of use, the produced callus cells or plant somatic embryos with human AP gene can be dried by any appropriate method.

The disclosed food product based on plants, containing human AP, may be used for internal use in dosed forms, including, but not limited to, as capsules, tablets, sachet bags and other dosage forms.

The disclosed food product based on plants, containing human AP, may be used as a component of food products for mass consumption, for example, dairy products, beverages, confections, as well as a component of medical and functional food products.

The disclosed product based on plant callus cells and somatic embryos, containing human AP, may be used for health maintenance, including for maintaining gastrointestinal tract (GI tract) homeostasis and for preventing diseases associated with the disruption of GI tract homeostasis. In particular, this product may be used to:
- regulate GI tract microflora, including maintaining normal GI tract microflora and preventing the growth of pathogenic microflora in the GI tract;
- regulate the immune system of the organism, including maintaining normal immune system activity and restoring it during immune system disorders, associated with different negative factors, including stress, environmental factors, use of medicines and other xenobiotics;
- alleviate toxic effects, including those associated with bacterial infection or inflammatory bowel diseases;
- prevent GI tract disorders, associated with the use of different xenobiotics, including medicines.

The food product based on plants, containing human AP, may be used by both healthy persons and persons that suffer from different inflammatory and autoimmune diseases, including bowel diseases (ulcerative colitis, Crohn's disease, enterocolitis), arthritis, eczema and other systemic diseases, associated with increased cell wall permeability. The food product also may be used by persons that suffer from obesity and different cosmetic problems.

Production of a food product with the disclosed method may be illustrated by the following examples.

### Example 1. Production of a food product based on carrot, containing human AP.

To introduce human intestinal AP gene into a plant expression vector, nucleotide sequence of recombinant gene of human AP with a size of 1587 base pairs was created by synthesis in full conformity with the nucleotide sequence of native mRNA of the human alkaline phosphatase gene. Then a sequence of restriction site *Bgl*II (agatct) was added into the sequence of recombinant AP gene at 5'-end before the translation initiation site (atg), while a sequence with the restriction site *Xba*I (atctagaat) was added at 3'-end after the stop codon (tga). The resulting nucleotide sequence with a size of 1602 base pairs was cloned into a plasmid pAL-T vector. Nucleotide sequence with a size of 1600 base pairs was removed from the resulting pAL-T-AP plasmid at restriction sites *Bgl*II and *Xba*I and ligated into an earlier created plasmid p35S-NLS-*rec*A-*lic*BM3, producing plasmid p35S-AP, in which the recombinant AP gene is under the control of 35S promoter of cauliflower mosaic virus. The accuracy of assembly of genetic construction p35S-AP was verified by sequencing.

Agrobacterial strain AGLO p35S-AP with human AP gene was produced using *Escherichia coli* cells with p35S-AP plasmid as donors, *E. coli* cells of HB101 pRK2013 strain as a facilitator of conjugative transfer and *Agrobacterium tumefaciens* cells of strain AGLO, as an acceptor.

Callus cells from zygotic embryos of carrot seeds were used as the object of agrobacterial transformation, which were produced as follows: carrot seeds were sterilized in a 50% aqueous solution of a chlorine-containing commercial disinfectant with the addition of Tween-20 (1 drop per 100 ml of solution), then washed three times in sterile distilled water for 10 minutes each time. The embryos were then extracted from the sterilized seeds in sterile conditions, which were then transferred into vials with MSM nutrient medium, enriched with growth regulators 2,4-D and kinetin. The vials were placed into thermostat and incubated in darkness at 23 °C until the callus formed.

Agrobacterial strain AGLO was produced as follows: in a laminar flow cabinet, separate bacterial colonies from a fresh bacterial culture with selective antibiotics were put into a vial (using a sterile inoculation loop) containing 3 of sterile liquid medium LB with composition indicated above. The agrobacteria were grown for 24-48 hours at 28 °C on a shaker with circular rotation (amplitude 5-10 cm and rate 150-200 RPM) equipped with thermostat.

Transformation and growing of carrot callus cells was performed as follows: carrot callus cells from zygotic embryos were placed on sterile filter paper in Petri dishes in a laminar flow cabinet, and 10-25 mcl of overnight culture of AGLO agrobacteria with alkaline phosphatase gene were applied on each transplant. Afterwards the callus was slightly dried and transported into vials onto MSM nutrition medium with 0.2-0.5 mg/L of 2,4-D. After 3 days the calluses were transported on an agar medium with the same composition but with the addition of 500 mg/L cefotaxime. Callus cultivation was performed for 10 days in darkness at 22-24 °C. Then 1-2 more transfers were performed onto a medium with the same composition until new callus cell colonies appeared. To reproduce them, callus cells were transferred onto MSM nutrition medium with 0.2-0.5 mg/L 2,4-D and 200 mg/L cefotaxime. Selection of transgenic carrot cells was performed using the ability of alkaline phosphatase to dephosphorylate para-nitrophenylphosphate, producing yellow-colored para-nitrophenol.

Carrot cells, containing human AP gene, were then washed with distilled water from the remaining nutrition medium and were freeze dried at -55 °C. The activity of alkaline phosphatase in the dried cell mass was determined by the ability of alkaline phosphatase to dephosphorylate para-nitrophenylphosphate; the dried mass was put into sachet bags.

The resulting food product was used to maintain normal GI tract microflora and normal condition of immune system, by adding it into dairy products or beverages, 0.5-1 sachet bag per person per day.

### Example 2. Production of a food product based on stevia, containing human AP.

The food product based on plants, containing recombinant human AP, was produced as described in Example 1, but using stevia (Stevia rebaudiana) as the production plant and somatic embryogenesis technology to grow AP-containing cells. To produce stevia somatic embryos, cell suspension was cultivated in liquid nutrition medium MSM, containing 0.2 mg/L indole-3-acetic acid and kinetin. Then, to obtain a homogenous population of somatic embryos, the suspension culture was filtered through nylon sieves with mesh size 120 mcm, then through 50 mcm. The cell mass that remained on the second sieve was transferred to a fresh medium to form embryos. On average, up to 70 thousand embryos could be produced from 1 L of the suspension.

The resulting food product was used to restore the disrupted immune system by adding it to food products or beverages that don't undergo heating above 40 °C, 0.5-1 sachet per person per day.

### Example 3. Production of a food product based on lettuce, containing human AP.

The food product based on plants, containing recombinant human AP, was produced as described in Example 1, but using lettuce (Latuca sativa) as the production plant and cloning human secretory AP gene into the plant expression vector, using GV3101 p35SAP strain as the agrobacterial strain, which was produced by using Escherichia coli cells with p35S-AP plasmid as donors, E. coli cells of HB101 pRK2013 strain as a facilitator of conjugative transfer and Agrobacterium tumefaciens cells of strain GV3101 as acceptors.

The resulting food product was used to alleviate the consequences of intoxications, including those associated with food poisoning, intestinal infections or medical drug use, by orally taking 1-2 sachets per person per day for 7-10 days.

### Example 4. Production of a food product based on Chinese cabbage, containing human AP.

The food product based on plants, containing recombinant human AP, was produced as described in Example 1, but using Chinese cabbage (Brassica pekinensis) as the production plant and MS cultivation medium with addition of 0.1-1.0 mg/L 2,4-D to produce and grow the callus cells.

The resulting food product was used to prevent negative adverse effects, associated with antibacterial drugs, by orally taking 1 sachet per person per day for 7-10 days.

### Example 5. Production of a food product based on cabbage, containing human AP.

The food product based on plants, containing recombinant human AP, was produced as described in Example 1, but using cabbage (Brassica oleracea) as the production plant and cloning human placental AP gene into the plant expression vector.

The resulting food product was used in volunteers to prevent negative adverse effects, associated with antibiotics use, by orally taking 1 sachet per person per day for 7-10 days.

### Example 6. Production of a food product based on dill, containing human AP.

The food product based on plants, containing recombinant human AP, was produced as described in Example 1, but using dill (Anethum graveolens) as the production plant and freeze-drying dill cells with human AP gene with finishing drying at a temperature of up to +30°C to produce the food product.

The resulting food product was used to prevent the disorders of GI tract and immune system, associated with negative action of stress factors, by orally taking 0.5-1 sachet per person per day for 7-10 days.

### Example 7. Production of a food product based on celery, containing human AP.

The food product based on plants, containing recombinant human AP, was produced as described in Example 1, but using celery (Apium graveolens) as the production plant and freeze-drying celery cells with human AP gene with finishing drying at a temperature of +30 to +40 °C to produce the food product.

The resulting food product was used to prevent the disorders of GI tract and immune system, associated with negative action of stress factors, by adding it to food products or beverages (that would not be heated above 40 °C), 0.5-1 sachet per person per day.

### Example 8. Production of a food product based on cucumber, containing human AP.

The food product based on plants, containing recombinant human AP, was produced as described in Example 1, but using cucumber (Cucumis sativus) as the production plant and B-5 cultivation medium with addition of 0.1-1.0 mg/L 2,4-D to produce and grow the callus cells.

### Example 9. Production of a food product based on squash, containing human AP.

The food product based on plants, containing recombinant human AP, was produced as described in Example 1, but using squash (Cucurbita pepo) as the production plant and mixing squash cells with human AP gene with excipients and putting them into gelatin capsules.

The resulting food product was used to prevent negative adverse effects, associated with antibacterial drugs, by orally taking 2 capsules per person per day for 7-10 days.

### Example 10. Production of a food product based on rice, containing human AP.

The food product based on plants, containing recombinant human AP, was produced as described in Example 1, but using rice (Oryza sativa) as the production plant and spray drying rice cells at a temperature of no more than +50 °C (to avoid protein denaturation) to produce the food product.

The resulting food product was used to prevent negative adverse effects, associated with antibiotics use, by orally taking 1-3 capsules per person per day for 7-10 days.

### Example 11. Production of a food product based on medicago, containing human AP.

The food product based on plants, containing recombinant human AP, was produced as described in Example 1, but using medicago (Medicago sativa) and synchronizing the embryos' development (by filtering them through sieves of different sizes, centrifuging and automatic separation) and additional growing of somatic embryos in a liquid nutrient medium with addition of PEG, mannitol or other compounds that increase the osmotic pressure.

### Example 12. Production of a food product based on tomato, containing human AP.

The food product based on plants, containing recombinant human AP, was produced as described in Example 1, but using tomato (Solanum lycopersicum) as the production plant and drying tomato cells at +40 °C to moisture content 4-8% and covering them with carboxymethyl cellulose and sodium alginate polymers, producing microcapsules/pellets, which were put into sachets or gelatin capsules to produce the final food product.

The resulting food product was used to prevent the disorders of GI tract and immune system, by adding it to food products or beverages, 0.5-1 sachet with microcapsules/pellets per person per day.

### Example 13. Restoration of immune parameters, disrupted by lipopolysaccharide action, using carrot cells, containing human AP gene.

Immune system disorders, associated with intestinal homeostasis disruption, in particular with the disruption of normal intestinal microflora and barrier functions, were modeled by orally administering E.coli lipopolysaccharide (Sigma-Aldrich) to CD-1 mice in 0.5 mg/kg dose.

LPS has increased the observed levels of proinflammatory cytokines IL-6, IL-8 and TNFα in murine blood, indicating the development of systemic inflammatory response. Anticipatory oral administration of carrot cells, containing human AP gene, to mice has considerably reduced the level of proinflammatory cytokines (by 53-77%).

### Example 14. Restoration of intestinal microflora after antibacterial treatment with streptomycin by feeding mice the carrot cells, containing human AP gene.

The ability of carrot, containing human intestinal AP gene, to restore intestinal microflora, disrupted after antibacterial therapy, was studied in CD-1 mice.

The first animal group was administered the antibiotic streptomycin once orally in 200 mg/kg dose, the second group - once orally streptomycin in 200 mg/kg dose and freeze-dried carrot cells, containing human intestinal AP gene, in 100 mg/kg dose; the third group - once orally streptomycin in 200 mg/kg dose and «Linex» probiotic drug, containing freeze-dried lactic acid bacteria (Lactobacillus acidophilus, Bifidobacterium infantis, Enterococcus faecium), in 100 mg (capsule mass)/kg dose.

Every day the mice feces were gathered and seeded on LB nutrition medium, and the presence or absence of normal intestinal microflora was examined (Table 6).

**Table 6 - Qualitative assay (presence of absence) of total count of intestinal microfloral bacteria in mice feces**

| Time (days) | Bacteria in mice feces | | |
|---|---|---|---|
| | Streptomycin | Streptomycin and freeze dried carrot cells, containing human intestinal AP gene | Streptomycin and «Linex» probiotic drug |
| 1 | Absent | Absent | Absent |
| 2 | Absent | Absent | Absent |
| 3 | Absent | Absent | Absent |
| 4 | Absent | Present | Absent |
| 5 | Absent | Present | Absent |
| 6 | Absent | Present | Absent |
| 7 | Absent | Present | Absent |
| 8 | Absent | Present | Present |
| 9 | Present | Present | Present |
| 10 | Present | Present | Present |

As the data demonstrates, in streptomycin-treated mice the growth of bacteria has halted 24 h after the use of the drug. The feces of this group of animals remained sterile for 8 days and started to restore only on Day 9. In mice group taking streptomycin and freeze dried carrot cells, containing human intestinal AP gene, the microflora started to restore after 4 days, whereas in mice group taking streptomycin and the reference drug «Linex», the microflora started to restore only after 8 days.

Thus, consumption of carrot cells, containing human intestinal AP gene, by mice improves the restoration of intestinal microflora after streptomycin treatment.

### Example 15. Carrot cells, containing human intestinal AP gene, promote the restoration of intestinal microflora after antibacterial treatment with ampicillin.

The experiments used CD-1 mice. The first animal group was administered the antibiotic ampicillin orally for 7 days in 50 mg/kg daily dose, the second group - ampicillin and freeze-dried carrot cells, containing human intestinal AP gene, in 50 mg/kg daily dose; the third group - ampicillin and «Linex» probiotic drug, containing freeze-dried lactic acid bacteria (Lactobacillus acidophilus, Bifidobacterium infantis, Enterococcus faecium), in 50 mg (capsule mass)/kg daily dose.

Every day for 3 weeks the mice feces were gathered and seeded on LB nutrition medium. The results are presented in Table 7.

**Table 7 - The presence of bacteria in the feces of ampicillin-treated mice and mice taking simultaneously ampicillin and freeze-dried carrot cells, containing human intestinal AP gene.**

| Time (days) | Bacteria in mice feces | | |
|---|---|---|---|
| | Ampicillin | Ampicillin and freeze-dried carrot cells, containing human intestinal AP gene | Streptomycin and «Linex» probiotic drug |
| 1 | Present | Present | Present |
| 2 | Absent | Absent | Absent |
| 3 | Absent | Absent | Absent |
| 4 | Absent | Absent | Absent |
| 5 | Absent | Absent | Absent |
| 6 | Absent | Absent | Absent |
| 7 | Absent | Absent | Absent |
| 8 | Absent | Absent | Absent |
| 9 | Absent | Absent | Absent |
| 10 | Absent | Absent | Absent |
| 11 | Absent | Present | Absent |
| 12 | Absent | Present | Absent |
| 13 | Absent | Present | Absent |
| 14 | Absent | Present | Absent |
| 15 | Absent | Present | Absent |
| 16 | Absent | Present | Absent |
| 17 | Absent | Present | Absent |
| 18 | Absent | Present | Present |
| 19 | Absent | Present | Present |
| 20 | Present | Present | Present |
| 21 | Present | Present | Present |

Bacterial growth in murine intestine has stopped 24 hours after the start of ampicillin treatment. In ampicillin group normal intestinal microflora started to restore only on Day 20 of the experiment. However, in the mice group taking ampicillin together with carrot cells, containing human intestinal AP gene, normal intestinal microflora has started to restore on Day 11 of the experiment. In mice group taking streptomycin and the reference drug «Linex», the microflora started to restore only on Day 18.

The disclosed invention may be widely used as a component of food products for mass consumption, for example, dairy products, beverages, confections, as well as a component of medical and functional food products.

### References

1. Bol-Schoenmakers M., Fiechter D., Raaben W., Hassing I., Bleumink R., Kruijswijk D., Maiioor K., Tersteeg-Zijderveld M., Brands R., Pieters R. Intestinal alkaline phosphatase contributes to the reduction of severe intestinal epithelial damage // Eur J Pharmacol. 2010/ - Vol. 633(1-3). - P.71-77. doi: 10.1016/j.ejphar.2010.01.023. Epub 2010 Feb 2.
2. Brenchley, J.M., Prince D.A., Schacker T.W., Ascher T.E., Silvestri G., Rao S., Kazzaz Z. et al. Microbial translocation is a cause of systemic immune activation in chronic HIV infection / // Nat. Med. - 2006. - Nº 1 Bird AP. CpG islands as gene markers in the vertebrate nucleus. Trends Genet 1987; 3: 342-7. 2. - P. 1365-1371. PMID: 17115046 DOI: 10.1038/nm1511.
3. Cadwell K., Patel K.K., Maloney N.S., Liu T.C., Ng A.C., Storer C.E., Head R.D., Xavier R., Stappenbeck T.S., Virgin H.W. Virus-plus-susceptibility gene interaction determines Crohn's disease gene Atg16L1 phenotypes in intestine // Cell. 2010. - Vol.141. - P.1135-1145. DOI: 10.1016/j.cell.2010.05.009.
4. Eskandari M.K., Kalff J.C., Billiar T. R., Lee K.K., Bauer A.J. LPS-induced muscularis macrophage Nathan CF. Secretory products of macrophages // J Clin Invest. 1987. - Vol. 79. - P. 319-326.
5. Jiang, W., Lederman M.M., Hunt P., Sieg S.F., Haley K., Rodriguez B., Landay A., Martin J., Sinclair E., Asher A.I., Deeks S.G., Douek D.C., Brenchley J.M. Plasma levels of bacterial DNA correlate with immune activation and the magnitude of immune restoration in persons with antiretroviral-treated HIV infection // J. Infect. Dis. - 2009. - Nº 199. - P. 1177-1185. PMID: 19265479 PMCID: PMC2728622 DOI: 10.1086/597476.
6. Kats S., Brands R., Hamad M.A.S.Seinen W.,Schamhorst V., Wulkan R. W., Schonberger J., P., van Oeveren W. Prophylactic treatment with cardiac surgery induces end phosphatase release // Int J Artif Organs 2012. Vol. 35. No 2. P. 144-151.
7. Maloy K.J., Powrie F. Intestinal homeostasis and its breakdown in inflammatory bowel disease // Nature. 2011. - Vol. 474. - P. 298-306. PMID: 2167774, DOI:10.1038/nature10208
8. Matzinger P. The danger model: a renewed sense of self // Science. 2002.- N. 12. Vol. 296(5566). - P. 301-305. PMID:11951032, DOI: 10.1126/science.1071059.
9. Molnar K., Vannay A., Szebeni B., Banki N.F, Sziksz E., Cseh A., Győrffy H., Lakatos P.L. , Papp M., Arató A., Veres G. Intestinal alkaline phosphatase in the colonic mucosa of children ith inflammatory bowel disease. // World J Gastroenterol 2012. - Vol. 18. - P. 3254-3259 [PMID: 22783049 DOI: 10.3748/wjg.v18.i25.3254].
10. Muginova S.V., Zhavoronkova A.M., Polyakov A.E., Shekhovtsova T.N. Application of alkaline phosphatases from different sources in pharmaceutical and clinical analysis for the determination of their cofactors; zinc and magnesium ions // Anal Sci 2007. - Vol. 23. - P. 357-363 [PMID: 17372382].
11. Odell J.T., Nagy F., Chua N.H. Identification of DNA sequences required for activity of the cauliflower mosaic virus 35S promoter // Nature. 1985. V. 313. P. 810-812.
12. Park B. S, Lee J. O. Recognition of lipopolysaccharide pattern by TLR4 complexes // Exp Mol Med. 2013. - Vol. 45. - P. 66. http://dx.doi.org/10.1038/emm .2013.97.
13. Pickkers P., Snellen F., Rogiers P., Bakker J., Jorens P., Meulenbelt J. , Spapen H., Tulleken J. E., Lins R., Ramael S., Bulitta M., . van der Hoeven J. G. Clinical pharmacology of exogenously administered alkaline phosphatase // Eur J Clin Pharmacol. 2009. Vol. 65. P. 393-402.
14. Podolsky DK. Inflammatory bowel disease. // N Engl J Med . 2002.- Vol. 347.-P.417-429.
15. Poelstra K, Bakker WW, Klok PA, et al. A physiologic function for alkaline phosphatase: Endotoxin detoxification. Lab Invest 1997. - Vol. 76(3). - P. 319-327. PMID: 9121115.
16. Rezende, A. A., J. M. Pizauro, et al. (1994). "Phosphodiesterase activity is a novel property of alkaline phosphatase from osseous plate." Biochem J. 1994. - Vol. 301 (Pt 2).- P. 517-522. PMCID: PMC1137111.
17. Riggle K.M., Rentea R.M., Welak S.R., Pritchard K.A., Jr, Oldham K.T., Gourlay D.M. Intestinal alkaline phosphatase prevents the systemic inflammatory response associated with necrotizing enterocolitis // J Surg Res. 2013. - Vol. 180. - P. 21-26. http://dx.doi.org/10.1016/j.jss.2012.10.042.
18. Sabalza M., Christou P., Capell T. Recombinant plant-derived pharmaceutical proteins: current technical and economic bottlenecks // Biotechnol Lett. - 2014. DOI 10.1007/s10529-014-1621-3.
19. Sharma A.K., Sharma M.K. Plants as bioreactors: Recent developments and emerging opportunities // Biotechnology Advances 2009. - Vol. 27. - P. 811-832.
20. Conley AJ, Joensuu JJ, Richman A, Menassa R Protein body-inducing fusions for high-level production and purification of recombinant proteins in plants. // Plant Biotechnol J. 2011. -Vol. 9. - P. 419-433.
21. Tuin A., Poelstra K., de Jager-Krikken A., Bok L., Raaben W., Velders M.P., Dijkstra G. Role of alkaline phosphatase in colitis in man and rats. Gut 2009; 58: 379-387 [PMID: 18852260 DOI: 10.1136/gut.2007.128868].
22. Weemaes A.M., Meijer D.K., Poelstra K.. Removal of phosphate from lipid A as a strategy to detoxify lipopolysaccharide // Shock. 2002. - Vol. 18. - P. 561-566. http://dx.doi.org/10.1097/00024382-200212000-00013.
23. Wise A.A., Liu Z., Binns A.N. Three methods for the introduction of foreign DNA into Agrobacterium // Agrobacterium Protocols. Springer, 2006. P. 43-54.
24. Komahin R.A., Komahina V.V., Zhuchenko A.A. 2007. Creation of genetic constructions, containing bacterial gene recA E. coli, to induce recombination in plants. Agricultural Biology. V. 3. P. 25-32.

## Claims

1. A method of producing transformed plant cells, containing recombinant human alkaline phosphatase, comprising the creation of a plant expression vector with human alkaline phosphatase gene, introduction of the plant expression vector with human alkaline phosphatase gene into agrobacterial strains, production of plant callus cells, agrobacterial transformation of callus cells using agrobacterial strains; production of somatic embryos from the transformed callus cells and growing the somatic embryos, containing human alkaline phosphatase gene, in a suspension culture.

2. A method of claim 1, different in that after agrobacterial transformation of callus cells, the transformed callus cells with human alkaline phosphatase gene are grown in a suspension culture.

3. A method of claim 1, different in that the grown transformed plant cells, containing human alkaline phosphatase gene, are dried.

4. A method of claim 1, different in that the recombinant human alkaline phosphatase is a tissue-specific alkaline phosphatase, e.g. intestinal and placental, or a tissue-nonspecific alkaline phosphatase.

5. A method of claim 1, different in that the development of embryos is synchronised.

6. A method of claim 1, different in that the somatic embryos are grown in a liquid nutrient medium with addition of compounds that increase the osmotic pressure, such as polyethylene glycol, mannitol and other compounds that can increase the osmotic pressure.

7. A method of claim 1, different in that the transformed plant cells, containing human alkaline phosphatase gene, are used as capsules, tablets, sachet bags and other forms, ready for consumption.

8. The use of transformed plant cells, produced using the method in claim 1, for prevention of disorders of gastrointestinal tract microflora and its restoration in toxic and stress conditions.

9. The use of transformed plant cells, produced using the method in claim 1, for prevention of immune system disorders, associated with disruption of intestinal microflora and intestinal barrier function, and restoring a disrupted immune system as an immunostimulating agent.
